# EUROPEAN PATENT SPECIFICATION

(11) **EP 1 165 184 B1**
(45) Date of publication and mention of the grant of the patent: **03.12.2003**
(21) Application number: 00926864.0
(22) Date of filing: 07.04.2000
(51) Int. Cl.: A61P 19/00, A61P 19/10, A61K 45/06, A61K 31/00, A61K 31/138, A61K 31/4535

(54) **ESTROGEN RECEPTOR-BETA ANTAGONISM AND BONE DISEASES**
ANTAGONISMUS DES ESTROGEN-REZEPTORS-BETA UND KNOCHENKRANKHEITEN
RECEPTEUR DES OESTROGENES ET LES OS

(30) Priority: 09.04.1999 GB 9908235; 14.07.1999 US 143716 P
(43) Date of publication of application: 02.01.2002
(73) Proprietor: KARO BIO AB, 141 57 Huddinge (SE)
(72) Inventor: ANDERSSON, Goran, Dep. of Pathology, 141 86 Huddinge (SE); OHLSSON, Claes, Dep. of Internal Medicine, 413 45 Goteborg (SE); GUSTAFSSON, Jan Ake, Dep. of Medical Nutrition, 141 57 Huddinge (SE)
(74) Representative: Tombling, Adrian George
(86) International application number: EP0003199
(87) International publication number: WO00061230

(56) References cited:
- WO-A-00/55137
- WO-A-00/59897
- WO-A-97/09348
- WO-A-98/03634
- WO-A-98/56812
- WO-A-99/11760
- DE-A- 19 839 115
- EVANS, GLENDA ET AL: "The effects of raloxifene on tibia histomorphometry in ovariectomized rats" ENDOCRINOLOGY (1994), 134(5), 2283-8 , XP000563692
- SEEMAN, E. ET AL: "Present and future of osteoporosis therapy" BONE (1995), 17(2, SUPPL.), 23S-9S , XP000652650
- GRASSER, W.A. ET AL: "Common mechanism for the estrogen agonist and antagonist activities of droloxifene" J. CELL. BIOCHEM. (1997), 65(2), 159-171 , XP000971988
- LABRIE, FERNAND ET AL: "EM-652 (SCH 57068), a third generation SERM acting as pure antiestrogen in the mammary gland and endometrium" J. STEROID BIOCHEM. MOL. BIOL. (1999), 69(1-6), 51-84 , XP000978361
- PATENT ABSTRACTS OF JAPAN vol. 2000, no. 01, 31 January 2000 (2000-01-31) & JP 11 292872 A (SUMITOMO CHEM CO LTD), 26 October 1999 (1999-10-26)
- BARKHEM ET AL: "Differential response of estrogen receptor alpha and estrogen receptor beta to partial estrogen agonists/antagonists" MOLECULAR PHARMACOLOGY, vol. 54, no. 1, July 1998 (1998-07), pages 105-111, XP000978331 cited in the application

## Description

This invention relates to *selecting* modulators *of* estrogen receptor beta (Erβ) *for use* in hormone replacement therapy for metabolic bone disease. ERβ antagonists and partial agonists *can be used* to increase cortical bone mineral content and cortical bone strength.

Estrogen deficiency is a major risk factor for development of osteoporosis in post-menopausal women. In particular, the condition is primarily caused by the severe decrease of serum estrogen levels after cessation of ovarian function. The absence of estrogen results in increased bone resorption and a progressive negative bone remodelling balance, leading to reduced trabecular and cortical bone mass and an increased risk of bone fracture.

Osteoporosis is generally preceded by a condition called "osteopenia". Osteopenia is considered to be a condition where the subject has a BMD value between 1 and 2.5 standard deviations (S.D.) below the young adult mean. In osteoporosis the BMD is more than 2.5 SD below the young adult mean. Osteoporosis is considered established when one or more bone fractures have occurred. In the present application, terms such as "treatment" or "prevention" or "minimising the risk of" of "osteoporosis" embrace treatment or prevention or minimising the risk of "osteopenia" or "established osteoporosis."

The absence of estrogen in post menopausal women results in an increase in bone turnover (Turner, R.T., *et al* (1994) *Endocr Rev* **15,** 275-300) and a negative bone remodelling balance, leading to bone loss and an increased fracture risk. The decrease in bone mass can be prevented by treatment with estrogens (Lindsay, R., *et al* (1976) *Lancet* **1,** 1038-1041). Although the bone preserving effect of estrogen replacement is indisputable (Turner *et al supra* (1994), the cellular mechanism of action for this hormone effect is unclear. Osteoblasts express estrogen receptors (Eriksen, E.F., *et al* (1988) *Science* **241,** 84-86, Komm, B.S., *et al* (1988) *Science* **241,** 81-84), suggesting a direct effect of estrogens on bone tissue. However, other, indirect effects of estrogens on bone cannot be excluded.

It has been reported that a man who was homozygous for an inactivating point mutation in the ERα gene demonstrated unfused growth plates and severe osteopenia (Smith, E.P., *et al* (1994) *N Engl JMed* **331,** 1056-1061). Based on this clinical finding it was assumed that ERα is important for growth plate closure and for adult bone metabolism at least in human males. In contrast, mice lacking a functional ERα had only minor skeletal abnormalities (Taki, M., *et al* (1997) *J Bone Miner Res* **12,** S460), suggesting that other mechanisms or receptors for estrogen action might be important during skeletal development of the mouse.

The presence of both estrogen receptors (ERα and β) in bone cells suggest that at least some estrogenic effects are directly acting on bone tissue.

The cloning of the novel estrogen receptor, ERβ, suggested that there may exist alternative mechanisms of action for estrogen (Kuiper, G.G., *et al* (1996) *Proc Natl Acad Sci U S A* **93,** 5925-5930). We and others have demonstrated that ERβ is expressed in growth plate chondrocytes and osteoblasts, indicating a possible role for ERβ in the regulation of longitudinal bone growth and/or adult bone metabolism (Onoe, Y., *et al* (1997) *Endocrinology* **138,** 4509-4512, Arts, J., Kuiper, G.G., *et al* (1997) *Endocrinology* **138,** 5067-5070, Vidal, O., *et al* (1999) *J Bone Miner Res* **14** 923-29 , Nilsson, L.O., *et al* (1999) *J Clin Endocrinol Metab* **84,** 370-373; Windahl Bone **26** 117-121 (2000)). However, the physiological role of ERβ in the regulation of growth and bone metabolism is still unknown.

The molecular mechanisms of action for ERα versus ERβ have recently been investigated. ERα and ERβ have almost identical DNA-binding domains and studies in vitro have demonstrated that the two receptors have similar affinities for estrogenic compounds (Kuiper, G.G. *et al* (1996) *Proc Natl Acad Sci U S A* **93,** 5925-5930, Kuiper; G.G., *et al* (1997) *Endocrinology* **138,** 863-870, Tremblay, G.B., *et al* (1997) *Mol Endocrinol* **11,** 353-365). The amino-acid sequence of ERβ differs from ERα in the N- and C-terminal trans-activating regions. Therefore the transcriptional activation mediated by ERβ may be distinct from that of ERα (Paech, K., *et al* (1997) *Science* **277,** 1508-1510). Considering the great similarities in ligand- and DNA- binding specificity it has been speculated that a differential tissue distribution of estrogen receptors may be important for mediating tissue specific responses to estrogens (Kuiper, G.G., and Gustafsson, J.A. (1997) *FEBS Lett* **410,** 87-90). Thus, the unique trans-activating domains of the two receptor subtypes, in combination with differential tissue-distribution, or differential cell-type distribution within a tissue, could be important factors to determine the estrogen response in target tissues.

We have recently generated mice devoid of functional ERβ protein and reported that ERβ is essential for normal ovulation efficiency, but is not essential for female or male sexual development, fertility, or lactation (Krege, J.H., *et al* (1998) *Proc Natl Acad Sci U S A* **95,** 15677-15682).

Mice lacking a functional ERβ receptor protein were generated by gene targeting (Krege, J.H. *et al* Proc. Natl Acad. Sci, (1998), **95,** 15677-15682. Studies aiming to determine the physiological role of ERβ on bone structure and bone mineral content were conducted using dual X-ray absorptiometry (DXA) and Peripheral Quantitative Computerized Tomography (pQCT). These measurements were applied to male and female 4-week-old (pre-pubertal) and 12-week-old (adult) mice of the wild-type (+/+) or mutant (-/-) genotypes. In the pre-pubertal mice, body weights, length and mineral content of long bones were indistinguishable between wild-type and mutant mice of either sex. Surprisingly, in adult female ERβ -/- mice, the body weight was increased, the femoral bones were longer and had an increased bone mineral content (DXA) compared to wild-type female mice of the same age. In fact, the bones of female ERβ -/- were masculinized with regard to the parameters analysed. This bone phenotypic alteration was not restricted to long bones, but was also noted in vertebral and calvarial bones. In the tibial long bone, which has almost completed its longitudinal growth before the onset of puberty, the increase in bone mineral content was still observable in the mutant compared to wild-type female adult mice, showing that increased bone mineral content was independent of longitudinal bone growth. It is well known that ovariectomy in young growing rodents results in a decreased trabecular bone mineral density but also in an increased radial growth of the cortical bone. Both these effects are reversed by treatment with estrogen. The increase in bone mineral content in ERβ (-/-) females was not due to increased trabecular bone mineral density but to an increased cross sectional cortical bone area associated with a radial bone growth. It appears that the cortical bone in adult female ERβ-/- mice demonstrated a loss of feminization, indicating that ERβ is essential for the endogenous estrogen effect on cortical bone (periosteal growth) in female mice.

When pQCT analysis was employed to distinguish whether the observed effects of ERβ depletion were affecting either trabecular or cortical bone mineral density, the results surprisingly showed that the increased bone mineral density was restricted to the cortical bone, and that the trabecular volumetric bone mineral density was not affected. This is unlike the effect of estrogen depletion during menopause or following oophorectomy, when the over-whelming effect is on trabecular bone mineral density. Measurements of cortical bone parameters showed that the bones of the female ERβ -/- mice, in comparison to their wild-type littermates, exhibited an increased periosteal as well as endosteal circumference, leading to increased cortical bone area. This increased cortical cross-sectional bone area was computed to result in a corresponding 30% increase in bone strength parameters, i.e cortical cross-sectional moment of inertia and the cortical moment of resistance. The biomechanic properties of ERβ -/- female bones may be further verified using e.g bending and torsion tests.

WO97/09348 (Karo Bio AB) discloses the cloning of ERβ receptor described in Kuiper, G. 1996 above. WO99/05170 (Yale University) discloses the amino-acid sequence and nucleotide sequence of another form of the ERβ gene and related protein sequences. WO98/56812 (Karo Bio AB) discloses certain ERα and ER β selective ligands. Barkhem, T. *et al* Molecular Pharmacology **54** 105 - 112 (1998) discloses the selectivity of certain compounds for different forms of the estrogen receptor. WO99/11760 (The Regents of the University of California) discloses methods of screening test compounds for differential ligand activation of ER-α and ERβ at AP1 sites.

*The present invention is as defined in the appended claims.*

Methods in accordance with the invention will now be described with reference to the accompanying drawings, Figures 1 to 2, in which:
Fig. 1 shows DXA measurements of bone parameters of excised tibias; and
Fig. 2 is a DXA scan of female (top) tibia (mid) and vertebrae (bottom) from ERβ -/- and wildtype (WT) mice.

In the following examples the methods set out below were used:

### Animals

The animals were maintained under standardized environmental conditions, with free access to food and water. Genotyping of tail DNA was performed at 4-5 weeks of age using PCR with partly different primers than previously described (Krege, J.H., *et al* (1998) *supra*. The primers used were one primer in intron 2 (βNHD4-25; 5,-AGAATGTTGCACTGCCCCTGCTGC-3,), one in intron 3 (C1wt-27; 5-GGAGTAGAAACAAGCAATCCAGACATC-3,) and one in the Neo cassette (Neo-25; 5,-GCAGCCTCTGTTCCACATACACTTC-3,). The PCR program used was: 95°C 2 min, (95°C 30 s, 64°C 1 min, 72°C 1 min) for 30 cycles, and 72°C 7 min. A 650 bp product (βNHD4-25 and C1wt-27) was amplified for the homozygous wild-type (+/+) mice, a 450bp (βNHD4-25 and Neo-25) product was amplified for the homozygous mutant (-/-) mice and both bands were amplified for the heterozygous (+/-) mice.

### Histological examination

Right femora were fixed in 4% paraformaldehyde, embedded in paraffin, sectioned and stained with Alcianblue/Van Gieson. The width of the growth plate was measured using an image-processing system (Easy Image, Bergströms Instrument, Stockholm, Sweden) coupled to a microscope. The average of 30 growth plate measurements (3 sections, 10 measurements/section) was calculated for each femur.

### Dual X-Ray Absorptiometry (DXA)

Areal Bone mineral density (Areal BMD; BMC/cm²) and bone mineral content (BMC) were measured with the Norland pDEXA Sabre (Fort Atkinson, WI) and the Sabre Research software (3.6).

*In vivo* measurements of animals were performed in order to determine total body, spine and cranium BMC (medium resolution scan with line spacing set at 0.05 cm). Three mice were analyzed at a time. A mouse which was sacrificed at the beginning of the experiment was included in all the scans as an internal standard in order to avoid inter-scan variations.

*Ex vivo* measurements of the left femur and tibiae and vertebrae L5 were performed on excised bones placed on a 1 cm thick Plexiglas table. All bones compared were measured in the same scan (high resolution scan with line spacing set at 0.01 cm).

### Peripheral Quantitative Computerized Tomography (pQCT)

Computerized tomography was performed with the Stratec pQCT XCT Research M (Norland, software version 5.4B) operating at a resolution of 70 µm (Rosen, H.N., *et al* (1995) *Calcif Tissue Int* **57,** 35-39).

*Mid-diaphyseal pQCT scans* of femora and tibias were performed to determine the cortical volumetric bone mineral density (volumetric BMD), the cortical cross sectional area, the periosteal circumference, the endosteal circumference, the moment of resistance and the cross sectional moment of inertia. The mid-diaphyseal region of femora and tibias in mice contains only cortical bone.

*Metaphyseal pQCT scans* of left femora and tibias were performed to measure trabecular volumetric BMD. The scan was positioned in the metaphysis at a distance from the distal growth plate corresponding to 4% of the total length of the femur (an area containing cortical as well as trabecular bone). The trabecular bone region was defined by setting an inner threshold to 400 mg/mm³. The inter assay coefficients of variation (CV) for the pQCT measurements were less than 2%.

It should be emphasized that the DXA technique gives the areal BMD whereas the pQCT gives the real/volumetric BMD. Thus, the DXA technique gives the mineral content per area and not per volume. Therefore, a factor regulating the outer dimensions of a bone, will affect the areal BMD (DXA) but not the volumetric BMD (pQCT).

### Body Weight and Dimensions of Bones

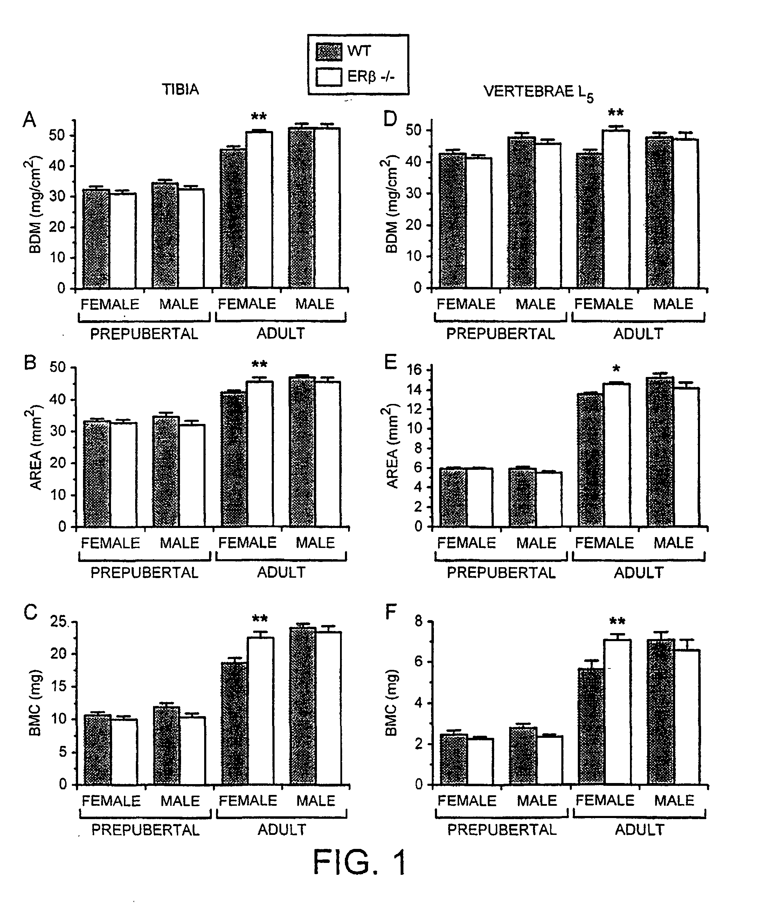

ERβ-/- and wild type mice were analyzed at 4 (Prepubertal) and 11 (Adult) weeks of age. The body weight, and length of the long bones were unchanged in prepubertal ERβ-/- mice compared with wt mice. No significant gender difference was seen between prepubertal wt females and wt males (t-test). However, adult male mice were, as expected, heavier and had longer tibias and femora than adult female mice (The results are shown in Table 1 and in Fig 1, t-test).

**Table 1**

| **Body Weight and Dimensions of Bones** | | | | | |
|---|---|---|---|---|---|
| | | **Female** | | **Male** | |
| | | WT | ERβ―/- | WT | ERβ―/- |
| **Prepubertal mice** | Body weight (g) | 14.8±0.5 | 14.7±0.6 | 16.7±0.6 | 16.1±0.4 |
| | | | | | |
| | Tibia length (mm) | 16.6±0.07 | 16.7±0.1 | 16.5±0.14 | 16.4±0.04 |
| | | | | | |
| | Femur length (mm) | 11.2±0.08 | 11.3±0.08 | 11.4±0.14 | 11.6±0.10 |
| | | | | | |
| **Adult mice** | Body weight (g) | 19.9±0.4 | 23.6±1.1** | 25.9±0.6 | 26.8±1.7 |
| | | | | | |
| | Tibia length (mm) | 17.6±0.11 | 17.9±0.11 | 18.2±0.04 | 18.0±0.16 |
| | | | | | |
| | Femur length (mm) | 14.6±0.13 | 15.2±0.11** | 15.0±0.09 | 15.2±0.20 |
| | | | | | |
| | Femur width (mm) | 1.6±0.19 | 1.83±0.13* | | |
| | Femur growth plate width (mm) | 123±8.1 | 106±5.6 | 99±6.1 | 106±11.1 |
| | | | | | |
| | Cortical thickness (mm) | 0.47±0.06 | 0.5±0.07 | | |
| | Growth plate height (µm) | 123± | 106±5.6 | | |
| | Growth plate volume density | 0.32±0.04 | 0.33±0.07 | | |

| | | | | | |
|---|---|---|---|---|---|
| 4 week old mice are indicated as prepubertal mice (n=6 for female WT and ER-/- mice; n=7 for male WT and ERβ -/- mice). 11 week old mice are indicated as adult mice (n=6/7 for female WT and ERβ -/- mice; n=5 for male WT and ERβ-/- mice). Femur growth plate width was measured in the distal femur. Values are given as means ±SEM. * p< 0.05, | | | | | |
| **p< 0.01 versus wild type (WT). | | | | | |

Specifically in the experiments illustrated by Fig 1 the area BMD (A, D), bone area (B, E) and BMC (C, F) of excised tibias (A-C) and vertebrae L5 (D-F) were measured using DXA technique as described in Methods. 4 week old mice are indicated as prepubertal 1mice (n=6 for female WT and ERβ-/- mice; n=7 for male WT and ERβ-/- mice). 11 week old mice are indicated as adult mice (n=7 for female WT and ERβ-/- mice; n=5 for male WT and ERβ-/- mice). Values are given as means±SEM. * p< 0,05, **p< 0,01 versus wild type (WT).

Surprisingly, the adult female ERβ-/- mice were heavier and had longer femora than adult female wt mice (Table 1).

A disproportional longitudinal bone growth, with a 4% increase in tibial and 30 % increase in femoral length, was seen from 4 weeks of age to 11 weeks of age in female wt mice (Table 1). Most likely this difference is due to the fact that most of the tibial growth occurs before puberty, whereas the femur demonstrates a significant pubertal/adult growthspurt. This observation provides a possible explanation to the finding of the present study that femoral but not tibial length is increased in ERβ deficient female mice and furthermore suggests that ERβ plays a repressive role during pubertal growth of murine females.

### Total Bone Mineral Content and Bone Mineral Content of Individual Bones

The bones from the ERβ-/- mice were analyzed in detail using DXA and pQCT techniques. The whole body BMC was increased in adult female but not in adult male ERβ-/- mice compared with wt mice as measured using DXA. The results are shown in Table 2.

**Table 2**

| **DXA Measurements of Bone Mineral Content *in vivo*** | | | | | | |
|---|---|---|---|---|---|---|
| | | | **Female** | | **Male** | |
| | | | WT | ERβ―/- | WT | ERβ―/- |
| **Prepubertal mice** | **Total body** | Bone mineral content (mg) | 367±18 | 334±11 | 418±13 | 393±17 |
| | Cranium | Bone mineral content (mg) | 140±4.0 | 123±4.1* | 145±3.4 | 138±3.8 |
| **Adult mice** | **Total body** | Bone mineral content (mg) | 507±15 | 596±15** | 603±20 | 626±34 |
| | **Cranium** | Bone mineral content (mg) | 201±2.9 | 234±5.9** | 204±4.0 | 215±6.1 |
| | **Spine** | Bone mineral content (mg) | 70±4.3 | 92±5.9** | 103±11 | 94±8.6 |

| | | | | | | |
|---|---|---|---|---|---|---|
| 4 week old mice are indicated as prepubertal mice (n=6 for female WT and ERβ -/- mice; n=7 for male WT and ERβ -/- mice). 11 week old mice are indicated as adult mice (n=7 for female WT and ERβ -/- mice; n=5 for male WT and ERβ -/- mice). Values are given as means ± SEM. * p< 0.05, | | | | | | |
| **p< 0.01 versus wild type (WT) | | | | | | |

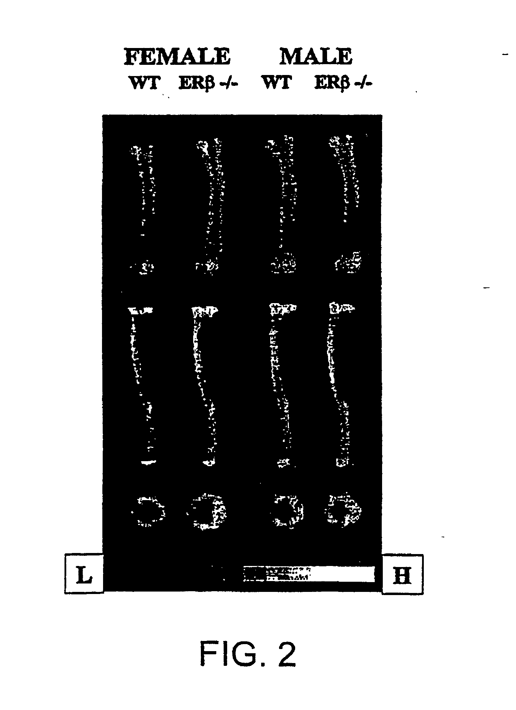

Actually, the gender difference seen in adult wt mice, with higher total BMC in males compared with females, was not seen in adult ERβ-/- mice (t-test). The effect on individual bones was also studied using DXA technique. The BMC of cranium, spine, vertebrae L5, tibia and femur were increased in adult female ERβ-/- mice compared with WT mice (Cranium 16%, spine 31 %, vertebrae L5 24%, tibia 20% and femur 27% over wt; Table 2, Fig 1-2). In Fig. 2 the relative areal BMD is indicated. (bottom; H= high density and L= low density). WT male mice had higher BMC than wt females in the spine, the vertebrae L5 and the tibia (Fig 1 and Table 2, t-test). This gender difference was not seen in ERβ-/mice. No difference in total-, spine-, tibial-and vertebral- BMC was seen between ERβ-/and wt in prepubertal male and female mice or in adult male mice.

The increase in tibial (20% over wt) and vertebral (24% over wt) BMC in adult female ERβ-/- mice was a result of both an increased bone area (Tibia 8%, vertebrae L5 11% over wt) and an increased areal BMD (Tibia 11%, vertebrae L5 16% over wt; Fig 1).

### Trabecular Bone Mineral Density

Trabecular volumetric BMD was measured in the metaphysis of the distal femur and proximal tibia using the pQCT technique. As seen in Table 3, adult but not prepubertal female mice had significantly lower trabecular volumetric BMD than male mice.

**Table 3**

| **Trabecular volumetric BMD and Cortical Bone Parameters as Measured using pQCT** | | | | | | |
|---|---|---|---|---|---|---|
| | | | **Female** | | **Male** | |
| | | | WT | ERβ―/- | WT | ERβ―/- |
| **Prepubertal** **mice** | **Tibia** | Trabecular density (mg/mm³) | 0.196±0.007 | 0.191±0.012 | 0.215±0.016 | 0.218±0.017 |
| | | Cortical density (mg/mm³) | 0.938±0.010 | 0.929±0.015 | 0.951±0.007 | 0.938±0.003 |
| | | Cortical area (mm²) | 0.48±0.03 | 0.46±0.02 | 0.52±0.02 | 0.54±0.01 |
| | | Cortical bone mineral content (mg/mm) | 0.46±0.03 | 0.43±0.03 | 0.49±0.02 | 0.50±0.01 |
| **Adult mice** | **Tibia** | Trabecular density (mg/mm³) | 0.282±0.011 | 0.299±0.009 | 0.331±0.006 | 0.328±0.007 |
| | | Cortical density (mg/mm³) | 1.130±0.010 | 1.172±0.004 | 1.168±0.011 | 1.173±0.015 |
| | | Cortical area (mm²) | 0.67±0.01 | 0.76±0.02** | 0.85±0.03 | 0.83±0.03 |
| | | Cortical bone mineral content (mg/mm) | 0.75±0.02 | 0.89±0.02** | 0.99±0.03 | 0.97±0.04 |
| | **Femur** | Trabecular density (mg/mm³) | 0.287±0.007 | 0.274±0.010 | 0.316±0.010 | 0.309±0.014 |
| | | Cortical density (mg/mm³) | 1.147±0.011 | 1.182±0.008* | 1.156±0.020 | 1.160±0.009 |
| | | Cortical area (mm²) | 0.80±0.02 | 0.91±0.03** | 0.97±0.03 | 0.99±0.05 |
| | | Cortical bone mineral content (mg/mm) | 0.91±0.03 | 1.07±0.04** | 1.12±0.04 | 1.15±0.06 |

| | | | | | | |
|---|---|---|---|---|---|---|
| 4 week old mice are indicated as prepubertal mice (n=6 for female WT and ERβ -/- mice; n=7 for male WT and ERβ -/- mice). 11 week old mice are indicated as adult mice (n=7 for female WT and ERβ -/- mice; n=5 for male WT and ERβ -/- mice). Values are given as means ± SEM. * p< 0.05, | | | | | | |
| **p< 0.01 versus wild type (WT) | | | | | | |

In contrast to the total BMC of tibia and femur no effect was seen on the trabecular volumetric BMD in adult female ERβ-/- mice compared with wt mice. Thus, the increased BMC in female ERβ -/- mice was not caused by an increased trabecular volumetric BMD.

### Cortical Bone Parameters

As the trabecular volumetric BMD was not changed the cortical bone parameters were studied in detail. Cortical bone parameters were investigated by mid-diaphyseal pQCT sections. The cortical BMCs of the mid-diaphyseal section of tibia and femur were increased in adult female ERβ-/- mice compared with wt (Tibia 19%, Femur 18% over wt) and this increase was mainly due to an increased cross-sectional bone area (Tibia 13%, Femur 14% over wt) and to a lesser extent an increased volumetric BMD (Tibia 4%, Femur 3% over wt; Table 3). As seen from Table 4, the increase in cross sectional area was associated with an increase of the periosteal circumference as well as the endosteal circumference.

**Table 4**

| **Mid-Diaphyseal Dimensions in Adult mice as Measured using pQCT** | | | | | |
|---|---|---|---|---|---|
| | | **Female** | | **Male** | |
| | | WT | ERβ―/- | WT | ERβ―/- |
| **Tibia** | **Cortical periosteal** circumference **(mm)** | 4.04±0.04 | 4.34±0.05** | 4.51±0.08 | 4.46±0.12 |
| | **Cortycal endosteal** circumference **(mm)** | 2.82±0.05 | 3.05±0.03** | 3.12±0.07 | 3.09±0.11 |
| | **Cortical cross sectional** moment of inertia **(mm**^{**4**}**)** | 0.17±0.01 | 0.24±0.02** | 0.29±0.02 | 0.29±0.03 |
| | **Cortical moment** of resistance **(mm**^{**3**}**)** | 0.23±0.01 | 0.29±0.01** | 0.32±0.02 | 0.30±0.02 |
| **Femur** | **Cortical periosteal** circumference **(mm)** | 4.93±0.07 | 5.28±0.06** | 5.52±0.16 | 5.51±0.10 |
| | **Cortical endosteal** circumference **(mm)** | 3.78±0.08 | 4.06±0.06* | 4.28±0.18 | 4.24±0.08 |
| | **Cortical cross** sectional moment of inertia **(mm**^{**4**}**)** | 0.33±0.02 | 0.46±0.02** | 0.54±0.05 | 0.56±0.05 |
| | **Cortical moment** of resistance **(mm**^{**3**}**)** | 0.36±0.01 | 0.46±0.02** | 0.49±0.03 | 0.50±0.03 |

| | | | | | |
|---|---|---|---|---|---|
| n=7 for female WT and ERβ-/- mice; n=5 for male WT and ERβ-/- mice. Values are given as means ± SEM. * p< 0.05, | | | | | |
| **p< 0.01 versus wild type (WT) | | | | | |

The changes in the size and position of the cross-sectional cortical bone area in adult female ERβ-/- mice resulted in a large increase of both the cortical cross sectional moment of inertia (Tibia 41%, Femur 39% over wt) and the cortical moment of resistance (Tibia 26%, Femur 28% over wt; Table 4). There was a gender difference for all of the cortical bone parameters between adult female wt and male wt mice. However, no gender difference was seen for these parameters between female ERβ-/- mice and male ERβ-/mice.

### Effect of ovariectomy in adult female mice

Female ERβ -/- mice were ovariectomised (ovx) at 13 weeks of age. The bone of the animals were analyzed 8 weeks later. Ovx decreased the uterine weight in both ERβ -/and wt mice, demonstrating that the ovx was complete (Table 5). Ovx decreased the BMC to the same extent in ERβ -/- mice as in wt mice (Table 5). This decrease in BMC was associated with a large decrease in trabecular volumetric BMD and a small decrease in cortical BMC.

**Table 5**

| **Effect of ovariectomy in adult female mice** | | | |
|---|---|---|---|
| | | Female | |
| | | WT | ERβ -/- |
| **Uterine Weight** | | -82±3%** | -80 ±2%** |
| **Cranium** | Total BMC | 09.0±4% | -7.9±2.6% |
| **Vertebrae L**_{**5**} | Total BMC | -21 ±3% | -27±6% |
| **Tibia** | Total BMC | -20±1%* | -26±4%** |
| **Femur** | Total BMC | -18 ±2%* | -17±4* |
| | Trabecular density | -36 ±5% | -27 ±5%** |
| | Cortical BMC | -18 ±3** | -21 ±3** |
| | Cortical density | -6 ±0.9%** | -4 ±0.4%** |
| | Cortical area | -13 ±3%** | -18 ±3%* |

| | | | |
|---|---|---|---|
| Values are given as % decrease compared with sham operated control and expressed as means ± **SEM** (n=5-6) **p< 0,05, **p< 0,01 versus sham operated mice. | | | |

### Expression of osteoblast-and osteoclast associated genes

To investigate molecular mechanisms behind the bone phenotypic alterations in adult female ERβ -/- mice, the expression of osteoblast-α 1 (I) collagen (Col. I), alkaline phosphatase (ALP), osteocalcin (OC)) and osteoclast-(tartrate-resistant acid phosphatase (TRAP), cathepsin K (cat K))-associated mRNAs was assessed by RT-PCR of RNA extracted from long bones from wild type (wt) and ERβ -/- mice (Fig. 3). The expression levels of these genes were generally higher, and not sexually differentiated, in prepubertal compared to adult wt mice, consistent with increased bone metabolism and turnover during rapid post-natal growth. In female adult ERβ -/- mice, the expression of the osteoblast-associated genes was however increased compared to age-matched wt female mice. As to the osteoclast-associated genes, no difference could be observed between adult ERβ -/- and wt female mice, although expression levels were higher in adult females compared to males.

### Comparison between adult (11 wk.) and Senescent (1 year) mice.

### Various bone parameters were measured in 11 week old (i.e. Adult) and in older, one year old mice using the techniques described above and including conventional histomorphological examination. The results are shown in the Table 6.

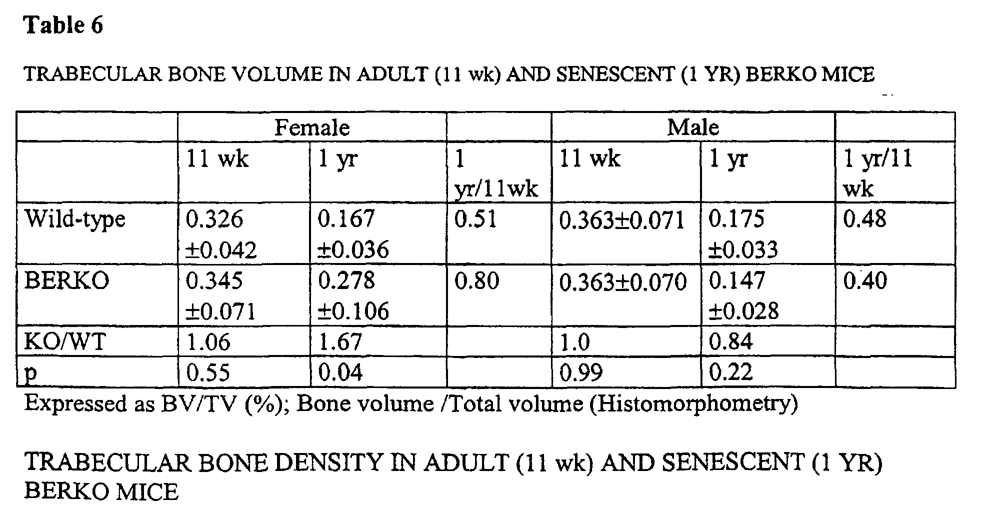

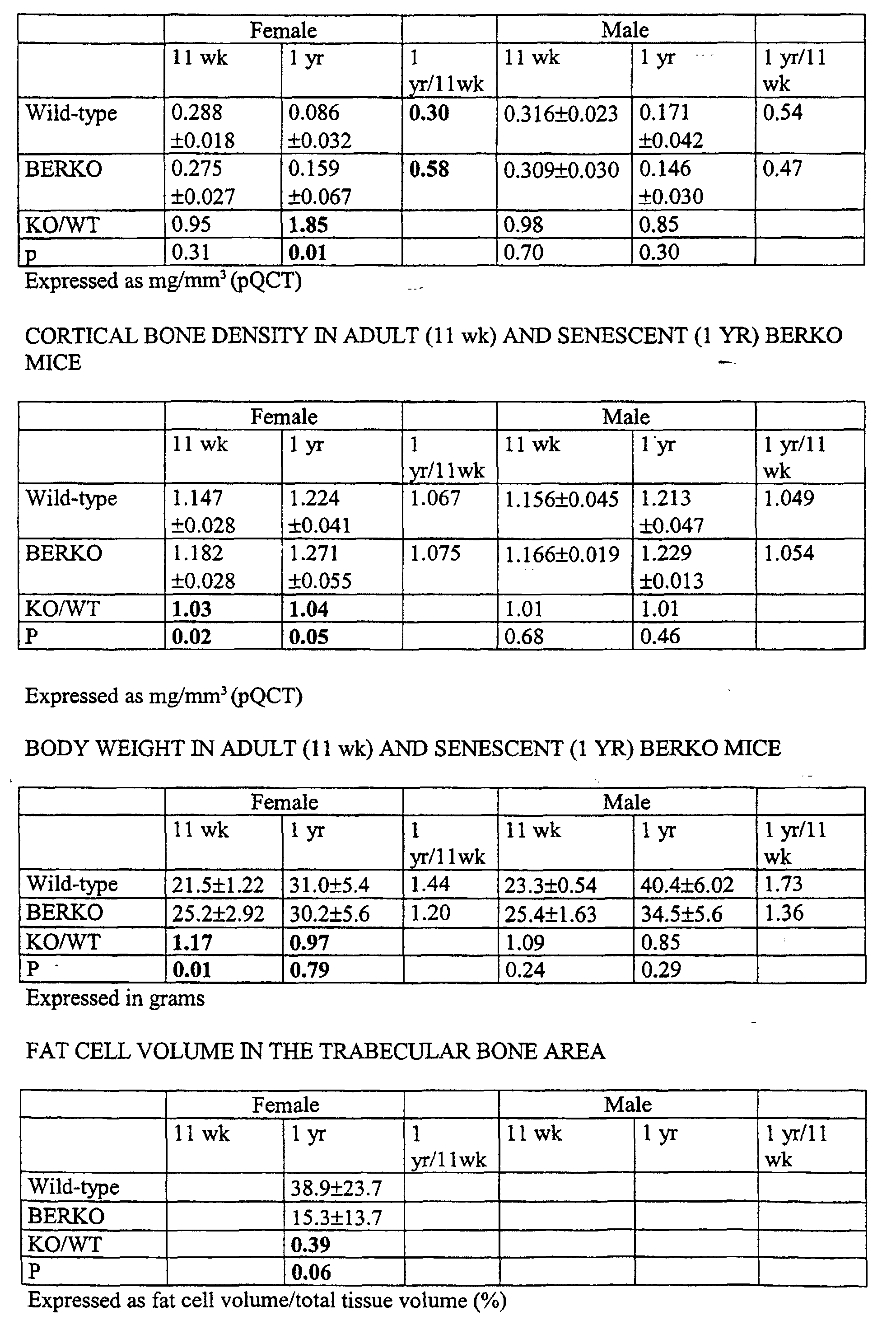

From these results it can be seen that the loss of bone trabecular mass in older mice is slowed down in female BERKO mice compared with wild-type female mice. There is also a good correlation between the histomorphological analysis and the other measurement techniques indicating the reliability of the latter techniques. Accordingly, an ERβ antagonist may be used to slow the natural reduction in bone trabecular mass in aging female mammals such as humans.

The present study demonstrated that the adult female ERβ -/- mice had an increased total BMC caused by increased cortical BMC while the trabecular volumetric BMD was unchanged. This increase in cortical BMC was mainly caused by an increase in the cortical cross-sectional area, associated with a radial cortical growth (increased periosteal circumference) of the bones. No effect was seen in male ERβ -/- mice. Actually, the gender differences seen in adult wt mice, with higher total BMC, increased cross-sectional cortical area and increased periosteal circumference in males compared with females, were not seen in adult ERβ-/- mice. Thus, it appears that the cortical bone in female ERβ-/- mice displays a loss of feminization. It has previously been demonstrated that gonadectomy of young rats results in decreased radial cortical bone growth in males and increased radial growth in females such that the sex difference is largely eliminated. The sex difference is reestablished in ovariectomized (Ovx) rats by administration of estrogen to females (Turner *et al* (1994) *supra*, Turner, R.T., *et al* (1987) *J Bone Miner Res* **2,** 115-122). This finding together with our present results of an apparent loss of feminization of the cortical bone in adult female ERβ -/- mice indicates that ERβ may be involved in feminization of the cortical bone in rodents. The effect of ERβ on cortical bone mass may include direct effects on the bone tissue or indirect effects including regulation of systemic hormones. A direct effect on the bone tissue is supported by recent findings that ERβ is expressed in osteoblasts (Onoe, Y., *et al* (1997) *Endocrinology* **138,** 4509-4512,Arts, J., Kuiper, G.G., *et al* (1997) *Endocrinology* **138,** 5067-5070,Vidal, O.,*et al* (1999) *J Bone Miner Res* ***supra***).In the tibial long bone, which has almost completed its longitudinal growth before the onset of puberty, an increase in BMC but not bone length was seen in the adult female ERβ -/- mice compared with wt mice. This finding demonstrates that the increased BMC in adult female ERβ -/- mice not only was caused by a stimulation of longitudinal bone growth.

It is well known from clinical and rat studies that Ovx results in a decrease in BMC and that this decrease predominantly is caused by a reduction of the trabecular volumetric BMD (Turner, R.T., *et al* (1994) *supra*). The Ovx mouse model is less well characterized. However, it was recently demonstrated that ovariaectomy in mice also results in a large decrease in trabecular volumetric BMD (Andersson, A., *et al* (1998) *Bone* **23**, S631). Interestingly, in the present study, no reduction in trabecular volumetric BMD was seen in ERβ-/- females compared with wt mice. These findings together indicate that Ovx-induced reduction in trabecular volumetric BMD is not solely dependent on ERβ. Therefore, one may speculate that either ERα by itself or that ERα and ERβ in a redundant manner protects against Ovx-induced trabecular bone loss. Future experiments with Ovx of ERα, ERβ and ERαβ double knockout mice will provide more insight to the receptor specificity of estrogens on bone mass. A functional receptor specificity for ERα versus ERβ in cardiovascular homeostatis has recently been described (Iafrati, M.D., *et al* (1997) *Nat Med* **3,** 545-548). It was demonstrated that estrogen inhibits vascular injury response by a mechanism that is independent of the ERα, suggesting that ERβ was involved in this response. Thus, ERα and ERβ may have both independent and common functions in normal physiology as well in pathophysiology.

In conclusion, ERβ -/- mice have unchanged trabecular BMD. In contrast, adult female mice devoid of ERβ have an increased cortical bone mineral content caused by an increased cross-sectional bone area, resulting in an apparent loss of feminization of the cortical bone. This finding indicates that ERβ is essential for the endogenous estrogen effect on cortical bone in female mice. The mechanism(s) of action for the effect of ERβ on cortical bone remains to be elucidated.

## Claims

1. A method of selecting compounds for the treatment or prevention of osteoporosis, the method comprising selecting a compound on the basis of its ability to antagonise agonist-dependent ERβ activity and wherein the effect of the compound on a bone-related parameter in a mammal is also determined.

2. A method according to claim 1 in which the bone-related parameter is selected from bone resorption, trabecular mass, cortical mass, reduction of fracture risk.

3. A method according to claim 2 and in which the bone-related parameter is bone resorption and in which compounds are selected on the basis of their ability to decrease bone resorption by at least 10 %.

4. A method according to claim 2 and in which the bone-related parameter is bone resorption and in which compounds are selected on the basis of their ability to increase trabecular mass by at least 3 %.

5. A method according to claim 2 and in which the bone-related parameter is bone resorption and in which compounds are selected on the basis of their ability to increase cortical mass by at least 3 %.

6. A method according to claim 2 and in which the bone-related parameter is bone resorption and in which compounds are selected on the basis of their ability to reduce the risk of bone fracture by at least 15 %.

7. A method according to any one of claims 1 to 6 in which the mammal is a mouse.

8. A method according to claim 7 in which the effect of the compound on a bone-related parameter in wild type mice is compared with the effect on the same parameter(s) in ERβ -/- mice.

## Patentansprüche

1. Verfahren zum Selektieren von Verbindungen zur Behandlung oder Vorbeugung von Osteoporose, wobei das Verfahren das Selektieren einer Verbindung auf der Grundlage ihrer Fähigkeit umfaßt, Agonisten-abhängige ERβ-Aktivität zu antagonisieren, und wobei ferner die Wirkung der Verbindung auf einen knochenbezogenen Parameter in einem Säuger bestimmt wird.

2. Verfahren nach Anspruch 1, bei dem der knochenbezogene Parameter ausgewählt ist aus Knochenresorption, trabekulärer Masse, kortikaler Masse und Senkung von Knochenbruchrisiko.

3. Verfahren nach Anspruch 2 und bei dem der knochenbezogene Parameter Knochenresorption ist und bei dem Verbindungen auf der Grundlage ihrer Fähigkeit selektiert werden, Knochenresorption um wenigstens 10% zu senken.

4. Verfahren nach Anspruch 2 und bei dem der knochenbezogene Parameter Knochenresorption ist und bei dem Verbindungen auf der Grundlage ihrer Fähigkeit selektiert werden, trabekuläre Masse um wenigstens 3% zu erhöhen.

5. Verfahren nach Anspruch 2 und bei dem der knochenbezogene Parameter Knochenresorption ist und bei dem Verbindungen auf der Grundlage ihrer Fähigkeit selektiert werden, kortikale Masse um wenigstens 3% zu erhöhen.

6. Verfahren nach Anspruch 2 und bei dem der knochenbezogene Parameter Knochenresorption ist und bei dem Verbindungen auf der Grundlage ihrer Fähigkeit selektiert werden, das Knochenbruchrisiko um wenigstens 15% zu senken.

7. Verfahren nach irgendeinem der Ansprüche 1 bis 6, bei dem der Säuger eine Maus ist.

8. Verfahren nach Anspruch 7, bei dem die Wirkung der Verbindung auf einen knochenbezogenen Parameter in Wildtyp-Mäusen mit deren Wirkung auf den oder die gleichen Parameter in ERβ -/- -Mäusen verglichen wird.

## Revendications

1. Procédé de sélection de composés pour le traitement ou la prévention de l'ostéoporose, le procédé comprenant la sélection d'un composé en fonction de sa capacité à antagoniser l'activité ERβ agoniste-dépendante et dans lequel l'effet du composé sur un paramètre lié à l'os chez un mammifère est également déterminé.

2. Procédé selon la revendication 1 dans lequel le paramètre lié à l'os est choisi parmi la résorption osseuse, la masse trabéculaire, la masse corticale ou la diminution du risque de fracture.

3. Procédé selon la revendication 2 et dans lequel le paramètre lié à l'os est la résorption osseuse et dans lequel les composés sont sélectionnés en fonction de leur capacité à réduire la résorption osseuse d'au moins 10 %.

4. Procédé selon la revendication 2 et dans lequel le paramètre lié à l'os est la résorption osseuse et dans lequel les composés sont sélectionnés en fonction de leur capacité à augmenter la masse trabéculaire d'au moins 3 %.

5. Procédé selon la revendication 2 et dans lequel le paramètre lié à l'os est la résorption osseuse et dans lequel les composés sont sélectionnés en fonction de leur capacité à augmenter la masse corticale d'au moins 3 %.

6. Procédé selon la revendication 2 et dans lequel le paramètre lié à l'os est la résorption osseuse et dans lequel les composés sont sélectionnés en fonction de leur capacité à diminuer le risque de fracture de l'os d'au moins 15 %.

7. Procédé selon l'une quelconque des revendications 1 à 6 dans lequel le mammifère est une souris.

8. Procédé selon la revendication 7 dans lequel l'effet du composé sur un paramètre lié à l'os chez des souris de type sauvage est comparé à l'effet sur les même(s) paramètre(s) chez des souris ERβ -/-.
